# EUROPEAN PATENT APPLICATION

(11) **EP 1 201 755 A1**
(43) Date of publication of application: **02.05.2002**
(21) Application number: 00949936.9
(22) Date of filing: 28.07.2000
(51) Int. Cl.: C12N 15/12, C07K 14/47

(54) **GENE OF HUMAN BRAIN TYPE PGT**

(30) Priority: 30.07.1999 JP 21784599
(71) Applicant: Mitsubishi Pharma Corporation, Osaka-shi, Osaka 541-0046 (JP)
(72) Inventor: KAWAMURA, Toru Mitsubishi Pharma Corporation, Chuo-ku, Tokyo 103-8405 (JP); HORIE, Satoshi Mitsubishi Pharma Corporation, Chuo-ku, Tokyo 103-8405 (JP); AKIRA, Toshiaki Mitsubishi Pharma Corporation, Chuo-ku,Tokyo 103 8405 (JP); UNO, Shusei Mitsubishi Pharma Corporation, Chuo-ku, Tokyo 103 8405 (JP)
(74) Representative: Forstmeyer, Dietmar, Dr. rer. nat., Dipl.-Chem.
(86) International application number: JP0005103
(87) International publication number: WO0109324

(57) **Abstract**

It is the matter to be solved by the present invention to provide mRNA coding for the PGT of a human brain type and to provide a polynucleotide coding for the PGT of a human brain type prepared using the said mRNA as a template. There are provided mRNA coding for the PGT of a human brain type and having a molecular weight of 38-40 kilodaltons and a polynucleotide being prepared by the use of the said mRNA as a template and having a specific base sequence represented by SEQ ID NO: 2 of the Sequence Listing.

## Description

### Technical Field

The present invention relates to a polynucleotide being concerned with a PGT (prostaglandin transporter) of a human brain type and also to a polypeptide corresponding to the sequence thereof. More particularly, it relates to mRNA coding for the PGT of a human brain type, to a polynucleotide prepared by using the said mRNA as a template and to a polypeptide corresponding to the sequence thereof.

### Background of the Invention

Prostaglandin transporter (PGT) has a property of transportation of prostaglandin compounds *in vivo* in a carrier-mediated manner. Prostaglandin permeates through a cellular membrane mediated by PGT to express a physiological activity in the cells thereof or to be metabolized/degraded by enzyme and the like. PGT has been known to be present in lung, kidney, brain, etc. in human body and, in addition, sequence of the gene coding for PGT has been confirmed as well (*Science*, Volume 268, pages 866-868, 1995). However, the PGT where its genetic sequence has been confirmed is that derived from lung while there has been no report for detailed functions and for a gene concerning a PGT of a brain type.

Further, according to the recent report, a relation of PGT with apoptosis (cell death) or particularly with apoptosis in neurons was pointed out (*J. Neurochemistry,* Volume 72, No. 5, pages 1907-1914, 1999) and participation of the PGT of a human brain type in diseases caused by apoptosis of neurons has been receiving public attention.

As a result of the rapid progress of genetic engineering in recent years, it is now possible that DNA coding for a desired protein is induced into vector by means of a recombination technique for DNA and then it is integrated with various microbes (such as *Escherichia coli,* yeast, *Bacillus subtilis,* etc.), animal cells and the like whereby tile said protein (physiologically active substance, various hormones, interferon, cytokine, vaccine, plasma protein, plasminogen activating factor, etc.) is produced in large quantities, in an efficient manner and in an industrial scale.

Clarification of the PGT of a brain type in a level of gene (RNA/DNA) using the above-mentioned means is the first step for the application of genetic engineering and recombination technique to the PGT of a brain type.

### Disclosure of the Invention

The matter to be solved by the present invention is to provide mRNA coding for the PGT of a human brain type and to provide DNA of a human brain type prepared by using the said mRNA as a template. More particularly, it is to provide mRNA coding for the PGT of a human type, to provide a polynucleotide of the PGT of a human brain type prepared by using the said mRNA as a template and to provide a polypeptide for which the said polynucleotide codes.

As a result of the progress of a study taking the above-mentioned circumstances into consideration, the present inventors have succeeded in collecting the mRNA coding for the PGT of a human brain type. They have further prepared the DNA of the PGT of a human brain type using the said mRNA as a template, analyzed its base sequence and found that it has an amino acid sequence consisting of a new sequence whereupon the present invention has been achieved.

The present invention relates to mRNA coding for the PGT of a human brain type, to a polynucleotide of the PGT of a human brain type prepared by using the mRNA as a template and to the corresponding polypeptide.

### Best Mode for Carrying Out the Invention

### (Collection of mRNA)

The mRNA of the present invention can be collected, for example, by such a manner that human brain cells are cultured, RNA is recovered from the cells having a high PGT-expressing activity, RNA having a poly A sequence is recovered from the said RNA and then the mRNA having the said poly A sequence is collected by a density-gradient centrifugation or the like.

It is also possible that the commercially available human brain poly A (+) RNA is purchased and the mRNA is collected therefrom. It is further possible that the desired mRNA is collected (hooked up) by using the already known gene of the PGT of a human lung type as a primer.

Size of the coding region of the said mRNA is about 1 kb.

### (Preparation of DNA)

The said DNA can be prepared by using the above-mentioned mRNA which codes for the PGT of a human brain type as a template. For example, an RNA-DNA hybrid is prepared from the mRNA coding for a human brain type using a reverse transcriptase and then mRNA is removed therefrom whereupon cDNA (complimentary DNA) is prepared. It is possible to prepare ds cDNA from that cDNA using polymerase. As a simple means, an RT-PCR (reverse transcription-polymerase chain reaction) or the like may be used.

The obtained product is transduced into a vector and, using the same, an appropriate host (such as *Escherichia coli)* is transformed. The transformed host (transformant) is cultured where positive colonies are selected. Then a plasmid DNA is recovered from the selected positive colonies whereupon the base sequence can be analyzed.

As to the base sequence, that which is represented by SEQ ID NO: 2 of the Sequence Listing may be exemplified.

The base sequence represented by SEQ ID NO: 2 of the Sequence Listing is a polynucleotide consisting of a base sequence of 1038 bases from 1(A) to 1038(A) having a homology to exons 1-3, GACG and exons 10-14 in the DNA coding for the PGT of a human lung type (SEQ ID NO: 1 of the Sequence Listing).

Incidentally, the relation between each exon of the PGT of a human lung type and the base sequence in SEQ ID NO: 1 of the Sequence Listing is shown as in Table 1.

**Table 1**

| Exon | Base Sequence (SEQ ID NO: 1) |
|---|---|
| 1 | 1 (A) ∼ 96 (G) |
| 2 | 97 (A) ∼ 234 (G) |
| 3 | 235 (A) ∼ 397 (G) |
| 4 | 398 (A) ∼ 625 (T) |
| 5 | 626 (C) ∼ 724 (G) |
| 6 | 725 (C) ∼ 861 (G) |
| 7 | 862 (A) ∼ 940 (C) |
| 8 | 941 (G) ∼ 1105 (G) |
| 9 | 1106 (G) ∼ 1295 (G) |
| 10 | 1296 (C) ∼ 1461 (G) |
| 11 | 1462(A)∼1626(T) |
| 12 | 1627(G)∼1695(G) |
| 13 | 1696(C)∼1814(G) |
| 14 | 1815 (G) ∼ 1932 (A) |

### (Polypeptide)

The polypeptide corresponding to the polynucleotide represented by SEQ ID NO: 2 of the Sequence Listing is represented by SEQ ID NO: 3 of the Sequence Listing. Difference of the said amino acid sequence from the PGT of a lung type is that a sequence of 298 amino acids from the 134th Asn to the 431st Pro in the PGT of a lung type is deficient. The amino acid sequence of the present invention is particularly characterized by the sequence of the 133rd Gly and the 134th Ser of SEQ ID NO: 3 of the Sequence Listing. Biological activity of the said PGT of the brain type can be detected by a method (a system where intracellular up-take of PGE1 is measured using HeLa cells) described in *Science*, Volume 268, pages 866-868, 1995.

### (Polynucleotide)

Besides the substance represented by SEQ ID NO: 2, the polynucleotide of the present invention further covers a complementary strand of that sequence and a polynucleotide which hybridizes to the polynucleotide represented by SEQ ID NO: 2 of the Sequence Listing under a stringent condition. Such a polynucleotide is useful as a probe or a primer for the detection of mRNA or gene coding for the PGT, as an antisense oligonucleotide for the regulation of expression of gene, etc.

### (Transformant)

The polypeptide of the present invention is provided by means of a genetic recombination technique by utilizing a cell-free protein synthesis system or a host known *per se* such as *Escherichia coli,* yeast, *Bacillus subtilis*, insect cells, animal cells, etc. Known methods *per se* are applied to the transformation and, for example, a transformation of host is carried out using plasmid, chromosome, virus, etc. as a replicon. Vector is selected depending upon the type of the selected host and its constituting elements are polynucleotide coding for a polypeptide with an object of expression and genetic sequence carrying the information concerning replication and control. Combination is different between prokaryotes and eukaryotes where promoter, binding site of ribosome, terminator, signal sequence, enhancer, etc. may be utilized and combined by a known method *per se.* Culture of the transformant is carried out by selecting the optimum condition for each host and, with regard to a marker, produced aimed peptide and/or transformant amount may be used.

Recovery of the polypeptide of the present invention from the culture medium may be carried out by a combination of molecular sieve chromatography (gel filtration) , ion exchange chromatography, affinity chromatography, etc. using a PGT activity as a marker or by a fractionating means depending upon the difference in solubility. Preferably, there is used a method where, depending upon the information of amino acid sequence, an antibody against the said sequence is prepared and a recovery by a specific adsorption by poly- or monoclonal antibody is carried out.

An antibody is prepared by selecting the antigenic determinant of the polypeptide of the present invention. The antigen is composed of at least 8 or more, preferably 10 or more or, more preferably, 12 or more amino acids. It is preferred that the antibody which is specific to the PGT of a brain type is characterized by the sequence of Gly and Ser which are 133rd and 134th in SEQ ID NO: 3 of the Sequence Listing. The selected antigen is carried out by conducting an immunological induction such as humoral response and/or cellular response to the animal either solely or bonding to a carrier in the presence or absence of adjuvant. There is no particular limitation for the carrier so far as the carrier *per* se does not cause a harmful action to the host and there may be exemplified cellulose, polymerized amino acid, albumin, etc. With regard to the animal to be immunized, there may be appropriately used mouse, rat, rabbit, goat, horse, etc. Polyclonal antibody is prepared by a method for the recovery of an antibody which is known *per se* while monoclonal antibody is prepared by such a method where antibody-producing cells are recovered from the animal which was subjected to an immunizing means and then a transformation means which is known *per se* to immortalized cells is introduced.

The polypeptide, polynucleotide and antibody prepared as such are useful as a marker for a diagnostic means in which the PGT of a human brain type is participated. When at least one of them is utilized, it is possible to construct a screening system for various medicaments. For example, a substance which inhibits the PGT is able to induce apoptosis of neurons. A substance which activates the PGT is able to suppress apoptosis of neurons. It is also possible by means of a screening to prepare a substance which has an affinity to the PGT and is incorporated into cells mediated by the PGT to suppress apoptosis. Accordingly, the polypeptide, polynucleotide and antibody of the present invention and a compound which is prepared by means of a screening utilizing them are useful for the diseases caused by apoptosis of neurons in which PGT is participated such as dementia.

### Examples

In order to illustrate the present invention in more detail, an example is given as hereunder although the present invention is never limited by that.

### Example:

About 1 kb of the product was recovered from a human brain poly A(+) RNA (manufactured by Clonetech) by an RT-PCR (reverse transcription polymerase chain reaction) using exon 1 and exon 14 primers of the PGT of a human lung type (DDBJ, U70867) of the already-issued paper (*Science*, Volume 68, pages 866-868, 1995) concerning cloning of the PGT gene of a human brain type. The recovered PCR product was integrated into a T-vector (PCR 2.1 vector; manufactured by Invitrogen), induced into *Escherlchia coli* (JM-109) and cultured. After that, a plasmid DNA was recovered from positive colonies and a base sequence was determined using a sequencer.

Reaction conditions for the RT-PCR were as follows.

With regard to the RT, primer and oligo dT were made to react under the following condition. Thus, 1) at 30°C for 10 minutes; 2) at 42°C for 30 minutes; 3) at 99°C for 5 minutes and then 4) at 5°C for 5 minutes.

With regard to the PCR, primer of each of exon 1 and exon 14 of the PGT of a human lung type (DDBJ, U70867) was used and the reaction was carried out under the following condition.

Exon 1-primer:

5′-TCCTGCCCAAGCTCGGCGTGTCCCAGGGCA-3′
(SEQ ID NO: 4 of the Sequence Listing)

Exon 14-primer:

5′-ACCCTCCAGCTGATGAAGCAAAGCAGCAGC-3′
(SEQ ID NO: 5 of the Sequence Listing)

Thus, 1) at 94°C for 2 minutes; 2) 40 cycles where each comprising at 94°C for 30 seconds, at 56°C for 30 seconds and 72°C for 1.5 minutes; and then 3) at 72°C for 10 minutes.

Incidentally, for the RT-PCR, there was used an RNA PCR kit (manufactured by Takara) and, for the duplication reaction, there was used a DNA thermal cycler (PJ 2000; manufactured by Perkin Elmer).

The DNA coding for the analyzed PGT of a human brain type was found to be a polynucleotide consisting of a base sequence as represented by SEQ ID NO: 2 of the Sequence Listing. This polynucleotide was able to encode the polypeptide consisting of 345 amino acid residues as represented by SEQ ID NO: 3 of the Sequence Listing.

Molecular weight as a protein of the PGT of a human brain type estimated from the amino acid sequence was roughly calculated whereupon it was 38-40 kilodaltons in the amino acid sequence of SEQ ID NO: 3 of the Sequence Listing.

### Industrial Applicability

mRNA and polynucleotide of a human brain type and polypeptide for which the said polynucleotide is coded according to the present invention are useful for clarification of action mechanism of the PGT in brain. Further, when the polynucleotide of the present invention is used, it is possible to produce the PGT of a human brain type by a genetic engineering means or, to be more specific, by a method for the transformation of appropriate host (e.g., microbes such as *Escherichia coli,* yeast, *Bacillus subtilis,* etc., animal cells and the like), a method for inducing into transgenic animals, etc. using the said polynucleotide

### Free Text of Sequence Listing

SEQ ID NO: 1 of the Sequence Listing: DNA coding for the PGT of a human lung type

SEQ ID NO: 2 of the Sequence Listing: DNA prepared using mRNA coding for the PGT of a human brain type as a template

SEQ ID NO: 3 of Sequence Listing: the PGT polypeptide of a human brain type estimated from the DNA of SEQ ID NO: 2

SEQ ID NO: 4 of Sequence Listing: a primer for the PCR derived from exon 1 of the PGT of a human lung type

SEQ ID NO: 5 of Sequence Listing: a primer for the PCR derived from exon 14 of the PGT of a human lung type

## Claims

1. mRNA which codes for a prostaglandin transporter (PGT) of a human brain type having a molecular weight of 38-40 kilodantons.

2. A polynucleotide selected from the following group:
1) a polynucleotide consisting of a base sequence represented by SEQ ID NO: 2 of the Sequence Listing;
2) a complementary strand of a polynucleotide consisting of a base sequence represented by SEQ ID NO: 2 of the Sequence Listing; and
3) a polynucleotide which hybridizes to a polynucleotide consisting of a base sequence represented by SEQ ID NO: 2 of the Sequence Listing under a stringent condition.

3. A polypeptide consisting of an amino acid sequence represented by SEQ ID NO: 3 of the Sequence Listing.


SEQUENCE LISTING

<110> MITSUBISHI PHARMA CORPORATIN

<120> Gene of human brain type PGT

<130> GP00-1016

<140> JP P1999-217845
<141> 1999-07-30

<160> 5

<170> PatentIn Ver. 2.1

<210> 1
<211> 1932
<212> DNA
<213> Homo sapiens

<400> 1
atggggctcc tgcccaagct cggcgtgtcc cagggcagcg acacctctac tagccgagcc 60
ggccgctgtg cccgctcggt cttcggcaac attaaggtgt ttgtgctctg ccaaggcctc 120
ctgcagctct gccaactcct gtacagcgcc tacttcaaga gcagcctcac caccattgag 180
aagcgctttg ggctctccag ttcttcatcg ggtctcattt ccagcttgaa tgagatcagc 240
aatgccatcc tcatcatctt tgtcagctac tttggcagcc gggtgcaccg tccacgtctg 300
attggcatcg gaggtctctt cctggctgca ggtgccttca tcctcaccct cccacacttc 360
ctctccgagc cctaccagta caccttggcc agcactggga acaacagccg cttgcaggcc 420
gagctctgcc agaagcattg gcaggacctg cctcccagta agtgccacag caccacccag 480
aacccccaga aggagaccag cagcatgtgg ggcctgatgg tggttgccca gctgctggct 540
ggcatcggga cagtgcctat tcagccattt gggatctcct atgtggatga cttctcagag 600
cccagcaact cgcccctgta catctccatc ttatttgcca tctctgtatt tggaccggct 660
ttcgggtacc tgctgggctc tatcatgctg cagatctttg tggactatgg cagggtcaac 720
acagctgcag ttaacttggt cccgggtgac ccccgatgga ttggagcctg gtggctaggc 780
ctgctcattt cttcagcttt attggttctc acctctttcc cctttttttt cttccctcga 840
gcaatgccca taggagcaaa gagggctcct gccacagcag atgaagcaag gaagttggag 900
gaggccaagt caagaggctc cctggtggat ttcattaaac ggtttccatg catctttctg 960
aggctcctga tgaactcact cttcgtcctg gtggtcctgg cccagtgcac cttctcctcc 1020
gtcattgctg gcctctccac cttcctcaac aagttcctgg agaagcagta tggcacctca 1080
gcagcctatg ccaacttcct cattggtgct gtgaacctcc ctgctgcagc cttggggatg 1140
ctgtttggag gaatcctcat gaagcgcttt gttttctctc tacaaaccat tccccgcata 1200
gctaccacca tcatcaccat ctccatgatc ctttgtgttc ctttgttctt catgggatgc 1260
tccaccccaa ctgtggccga agtctacccc cctagcacat caagttctat acatccgcag 1320
tctcctgcct gccgcaggga ctgctcgtgc ccagattcta tcttccaccc ggtctgtgga 1380
gacaatggaa tcgagtacct ctccccttgc catgccggct gcagcaacat caacatgagc 1440
tctgcaacct ccaagcaact gatctatttg aactgcagct gtgtgaccgg gggatccgct 1500
tcagcaaaga caggatcgtg ccctgtcccc tgtgcccact tcctgctccc ggccatcttc 1560
ctcatctcct tcgtgtccct gatagcctgc atctcccaca accccctcta catgatggtt 1620

ctgcgtgtgg tgaaccagga ggaaaagtca tttgccatcg gggtgcagtt cttgttgatg 1680
cgcttgctgg cctggctgcc atctccagcc ctctatggcc tcaccattga ccactcctgc 1740
atccggtgga actcgctgtg cttggggagg cgaggggcct gcgcctacta tgacaacgat 1800
gctctccgag acaggtacct gggcctgcag atgggctaca aggcgctggg catgctgctg 1860
ctttgcttca tcagctggag ggtgaagaag aacaaggagt acaacgtgca gaaggcggca 1920
ggcctcatct ga 1932

<210> 2
<211> 1038
<212> DNA
<213> Homo sapiens

<400> 2
atggggctcc tgcccaagct cggcgtgtcc cagggcagcg acacctctac tagccgagcc 60
ggccgctgtg cccgctcggt cttcggcaac attaaggtgt ttgtgctctg ccaaggcctc 120
ctgcagctct gccaactcct gtacagcgcc tacttcaaga gcagcctcac caccattgag 180
aagcgctttg ggctctccag ttcttcatcg ggtctcattt ccagcttgaa tgagatcagc 240
aatgccatcc tcatcatctt tgtcagctac tttggcagcc gggtgcaccg tccacgtctg 300
attggcatcg gaggtctctt cctggctgca ggtgccttca tcctcaccct cccacacttc 360
ctctccgagc cctaccagta caccttggcc agcactggca gcacatcaag ttctatacat 420
ccgcagtctc ctgcctgccg cagggactgc tcgtgcccag attctatctt ccacccggtc 480
tgtggagaca atggaatcga gtacctctcc ccttgccatg ccggctgcag caacatcaac 540
atgagctctg caacctccaa gcaactgatc tatttgaact gcagctgtgt gaccggggga 600
tccgcttcag caaaggcagg atcgtgccct gtcccctgtg cccacttcct gctcccggcc 660
atcttcctca tctccttcgt gtccctgata gcctgcatct cccacaaccc cctctacatg 720
atggttctgc gtgtggtgaa ccaggaggaa aagtcatttg ccatcggggt gcagttcttg 780
ttgatgcgct tgctggcctg gctgccatct ccagccctct atggcctcac cattgaccac 840
tcctgcatcc ggtggaactc gctgtgcttg gggaggcgag gggcctgcgc ctactatgac 900
aacgatgctc tccgagacag gtacctgggc ctgcagatgg gctacaaggc gctgggcatg 960
ctgctgcttt gcttcatcag ctggagggtg aagaagaaca aggagtacaa cgtgcagaag 1020
gcggcaggcc tcatctga 1038

<210> 3
<211> 345
<212> PRT
<213> Homo sapiens

<400> 3
Met Gly Leu Leu Pro Lys Leu Gly Val Ser Gln Gly Ser Asp Thr Ser
1               5                   10                  15

Thr Ser Arg Ala Gly Arg Cys Ala Arg Ser Val Phe Gly Asn Ile Lys
            20                  25                  30

Val Phe Val Leu Cys Gln Gly Leu Leu Gln Leu Cys Gln Leu Leu Tyr
        35                  40                  45

Ser Ala Tyr Phe Lys Ser Ser Leu Thr Thr Ile Glu Lys Arg Phe Gly
    50                  55                  60

Leu Ser Ser Ser Ser Ser Gly Leu Ile Ser Ser Leu Asn Glu Ile Ser
65                  70                  75                  80

Asn Ala Ile Leu Ile Ile Phe Val Ser Tyr Phe Gly Ser Arg Val His
                85                  90                  95

Arg Pro Arg Leu Ile Gly Ile Gly Gly Leu Phe Leu Ala Ala Gly Ala
            100                 105                 110

Phe Ile Leu Thr Leu Pro His Phe Leu Ser Glu Pro Tyr Gln Tyr Thr
        115                 120                 125

Leu Ala Ser Thr Gly Ser Thr Ser Ser Ser Ile His Pro Gln Ser Pro
    130                 135                 140

Ala Cys Arg Arg Asp Cys Ser Cys Pro Asp Ser Ile Phe His Pro Val
145                 150                 155                 160

Cys Gly Asp Asn Gly Ile Glu Tyr Leu Ser Pro Cys His Ala Gly Cys
                165                 170                 175

Ser Asn Ile Asn Met Ser Ser Ala Thr Ser Lys Gln Leu Ile Tyr Leu
            180                 185                 190

Asn Cys Ser Cys Val Thr Gly Gly Ser Ala Ser Ala Lys Ala Gly Ser
        195                 200                 205

Cys Pro Val Pro Cys Ala His Phe Leu Leu Pro Ala Ile Phe Leu Ile
    210                 215                 220

Ser Phe Val Ser Leu Ile Ala Cys Ile Ser His Asn Pro Leu Tyr Met
225                 230                 235                 240

Met Val Leu Arg Val Val Asn Gln Glu Glu Lys Ser Phe Ala Ile Gly
                245                 250                 255

Val Gln Phe Leu Leu Met Arg Leu Leu Ala Trp Leu Pro Ser Pro Ala
            260                 265                 270

Leu Tyr Gly Leu Thr Ile Asp His Ser Cys Ile Arg Trp Asn Ser Leu
        275                 280                 285

Cys Leu Gly Arg Arg Gly Ala Cys Ala Tyr Tyr Asp Asn Asp Ala Leu
    290                 295                 300

Arg Asp Arg Tyr Leu Gly Leu Gln Met Gly Tyr Lys Ala Leu Gly Met
305                 310                 315                 320

Leu Leu Leu Cys Phe Ile Ser Trp Arg Val Lys Lys Asn Lys Glu Tyr
                325                 330                 335

Asn Val Gln Lys Ala Ala Gly Leu Ile
            340                 345

<210> 4
<211> 30
<212> DNA
<213> Artificial Sequence

<220>
<223> Description of Artificial Sequence:Primer of exon
      1 derived from human lung PGT for PCR test

<400> 4
tcctgcccaa gctcggcgtg tcccagggca                                    30

<210> 5
<211> 30
<212> DNA
<213> Artificial Sequence

<220>
<223> Description of Artificial Sequence:Primer of exon
      14 derived from human lung type PGT for PCR test

<400> 5
accctccagc tgatgaagca aagcagcagc                                    30